(19) Europäisches Patentamt
European Patent Office
Office européen des brevets

(11) Publication number: 0 373 531 A1

# (12) EUROPEAN PATENT APPLICATION

(21) Application number: 89122685.4

(22) Date of filing: 08.12.89

(51) Int. Cl.[5]: C07D 498/06, A61K 31/535, //(C07D498/06,265:00,221:00)

Claims for the following Contracting State: ES.

(30) Priority: 12.12.88 JP 313298/88

(43) Date of publication of application: 20.06.90 Bulletin 90/25

(84) Designated Contracting States: DE ES FR GB IT

(71) Applicant: WAKUNAGA SEIYAKU KABUSHIKI KAISHA
1-39 Fukushima 3-chome Fukushima-ku
Osaka-shi Osaka 553(JP)

(72) Inventor: Yazaki, Akira c/o Wakunaga Seiyaku K.K.
Chuo Kenkyusho 1624, Shimo Kotachi
Koda-cho
Takata-gun Hiroshima(JP)
Inventor: Inoue, Satoshi c/o Wakunaga Seiyaku K.K.
Chuo Kenkyusho 1624, Shimo Kotachi
Koda-cho
Takata-gun Hiroshima(JP)
Inventor: Amano, Hirotaka c/o Wakunaga Seiyaku K.K.
Chuo Kenkyusho 1624, Shimo Kotachi
Koda-cho
Takata-gun Hiroshima(JP)

(74) Representative: Wächtershäuser, Günter, Dr.
Tal 29
D-8000 München 2(DE)

## (54) Novel pyridobenzoxazine derivative.

(57) Pyridobenzoxazine derivatives having a [3,2,1-ij][3,1]benzoxazine structure represented by the formula (I) and their salts are disclosed.

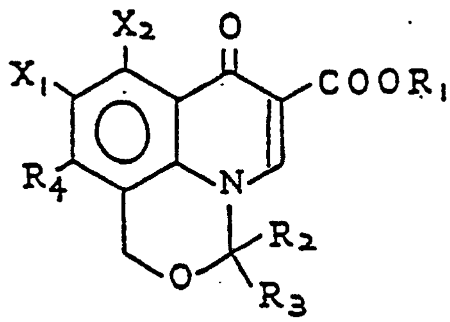

(I)

There are many varieties for the compound depending on the types of residues $R_1$-$R_4$, $X_1$, and $X_2$. The compounds of formula (I) and their salts have excellent antimicrobial activities and wide antimicrobial spectra, and are effective against both gram positive and gram negative microorganisms. They can be used as a medicine, an agrichemical, and a food preservative.

EP 0 373 531 A1

# NOVEL PYRIDOBENZOXAZINE DERIVATIVE

## BACKGROUND OF THE INVENTION

### Field of the Invention:

This invention relates to novel pyridobenzoxazine derivatives and their salts which are excellent synthetic antimicrobial substances.

### Description of the Background:

A number of compounds having a pyridonecarboxylic acid basic structure have been used as pharmaceuticals in recent years because of their excellent antimicrobial capability and a wide antimicrobial spectrum. Among these compounds, ofloxacin having a [1,2,3-de][1,4]benzoxazine structure (Japanese Patent Publication No.11955/1986) is highly evaluated as a medicine exhibiting both high antimicrobial capability and a superior absorption ratio. Generally, physicochemical characteristics of chemical compounds depend largely upon their chemical structure. This applies also to antimicrobial chemical compounds, and for this reason, constant, ever-lasting desire exists for new compounds having antimicrobial activities. In view of this situation, the present inventors have synthesized compounds having a [3,2,1-ij]-[3,1]benzoxazine structure and found that these compounds exhibited extremely strong antimicrobial activity. This finding has led to the completion of this invention.

## SUMMARY OF THE INVENTION

Accordingly, an object of this invention is to provide pyridobenzoxazine derivatives represented by the formula (I) or salts thereof.

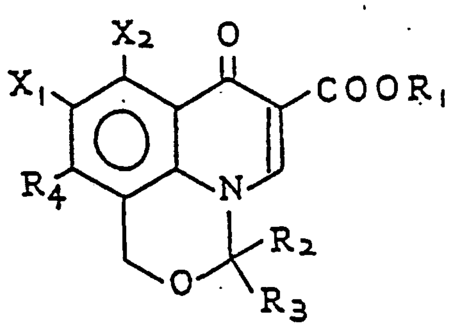

(I)

Various substituents in formula (I) have the following meanings:

$X_1$ and $X_2$: individually represent a hydrogen atom or a halogen atom.

$R_1$: represents a hydrogen atom or a carboxyl protective group.

$R_2$ and $R_3$: individually represent a hydrogen atom, a lower alkyl group which may be substituted by halogen atoms, or a lower alkenyl group; or $R_2$ and $R_3$ may, in combination, form a 3-6 membered ring together with the adjacent carbon atom.

$R_4$: represents a hydrogen atom, a hydroxyl group, a group $R_5$-O, wherein $R_5$ represents a lower alkyl group which may have a substituent, a cyclo-lower alkyl group which may have a substituent, an aryl group which may have a substituent, a 5 or 6 membered heterocyclic group which may have a substituent, or an alkyl- or arylsulfonyl group which may have a substituent; a group $R_6$S-, wherein $R_6$ represents a lower alkyl group which may have a substituent, a cyclo-lower alkyl group which may have a substituent, an aryl group which may have a substituent, or a 5 or 6 membered heterocyclic group which may have a substituent; or a group $R_7R_8$N-, wherein $R_7$ and $R_8$ individually represent a hydrogen atom, a lower alkyl

group which may have a substituent, a lower alkenyl group which may have a substituent, a cyclo-lower alkyl group which may have a substituent, a lower aralkyl group which may have a substituent, an aryl group which may have a substituent, a 5 or 6 membered heterocyclic group which may have a substituent, or $R_7$ and $R_8$ may, in combination, form a 4-7 membered cyclic amino group together with the adjacent nitrogen atom; provided (i) that the carbon atoms of the cyclic amino group may have one or more substituents selected from the group consisting of halogen atom, hydroxyl group, alkoxy group, acyloxy group, carboxyl group, alkoxycarbonyl group, aminocarbonyl group which may have a substituent, cyano group, mercapto group, alkylthio group, amino group which may have a substituent, acylamino group which may have a substituent, alkoxycarbonylamino group which may have a substituent, lower alkyl group which may have a substituent, lower alkenyl group which may have a substituent, cyclo-lower alkyl group which may have a substituent, aralkyl group which may have a substituent, and aryl group which may have a substituent, provided that when carbon atoms have two or more substituents which are lower alkyl groups which may have a substituent, the two lower alkyl groups which may have a substituent and the cyclic amino group may together form a bi-cyclic amino group; (ii) that said cyclic amino group or said bi-cyclic amino group may contain in their rings a group $-NR_9$, $-\underset{\underset{O}{\|}}{C}-$, -O-, -S-, -SO-, or $-SO_2-$; wherein $R_9$ is a hydrogen atom, a hydroxyl group, lower alkyl group which may have a substituent, lower alkenyl group which may have a substituent, cyclo-lower alkyl group which may have a substituent, aralkyl group which may have a substituent, aryl group which may have a substituent, acyl group which may have a substituent, a lower alkyloxycarbonyl group which may have a substituent, aralkyloxycarbonyl group which may have a substituent, alkylsulfonyl group which may have a substituent, or arylsulfonyl group which may have a substituent; provided that when $-NR_9-$ is contained in the ring, $R_9$ is a lower alkyl group which may have a substituent, and at least one lower alkyl group which may have a substituent is present on a carbon atom or carbon atoms of the cyclic amino group, one of the lower alkyl groups and said cyclic amino group which is represented by $R_7R_8N-$ may together form a bi-cyclic amino group, and (iii) further that said cyclic amino group or said bi-cyclic amino group may contain therein a double bond.

Other objects, features and advantages of the invention will hereinafter become more readily apparent from the following description.

## DETAILED DESCRIPTION OF THE INVENTION AND PREFERRED EMBODIMENTS

The compounds of formula (I) and their salts are novel compounds and exhibit a superior antimicrobial activities against both gram positive and gram negative microorganisms. The compounds of formula (I) and their salts therefore have a great value as antimicrobial agents. They can be used as a medicine for curing diseases caused by bacteria not only of man, but also of animals and fish.

Pyridobenzoxazine derivatives of the present invention have a general formula:

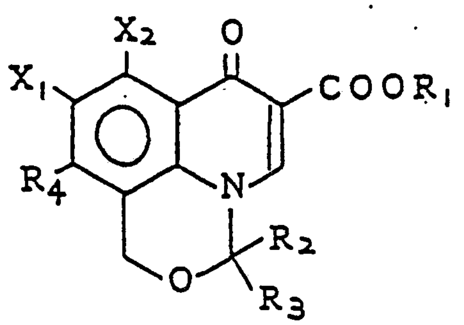

(I)

wherein $X_1$, $X_2$, and $R_1$-$R_4$ have the meanings defined above.

In the above definitions, "lower" means $C_1$-$C_7$, preferably $C_1$-$C_4$, when the group in question is a linear or branched group, and $C_3$-$C_7$ when the group is a cyclic group.

When $R_2$ and $R_3$ are different from each other, the carbon atom to which $R_2$ and $R_3$ are bonded is asymmetrical, and therefore there can exist optically isometric compounds of formula (I). The present invention includes each of such optical isomers as well as mixtures of such optical isomers.

Each substituent of the compound of formula (I) is now discussed in detail.

(a) $X_1$ and $X_2$

Halogen atoms in this invention include fluorine, chlorine, bromine, and iodine atoms. Fluorine atom is a preferable halogen atom.

(b) $R_1$

A carboxyl protective group means an ester residue of carboxylic acid ester. It may be a lower alkyl group, e.g. methyl, ethyl, n-propyl, isopropyl, or t-butyl groups; or any group which hydrolyzes in a living body and produces a free carboxylic acid such as lower alkanoyloxyxalkyl group (e.g. acetoxymethyl, 1-acetoxyethyl, or pivaloyloxymethyl group), lower alkoxycarbonyloxyalkyl (e.g. methoxycarbonyloxymethyl group or 1-ethoxycarbonyloxymethyl group), lactonyl group (e.g. phthalidyl group or thiophthalidyl group), lower alkoxymethyl group (e.g. methoxymethyl group), (5-methyl-2-oxo-1,3-dioxol-4-yl)methyl group, or the like.

(c) $R_2$ and $R_3$

A lower alkyl group which may be substituted by halogen atoms means a lower alkyl group which may be substituted by one or more halogen atoms, and includes such groups as methyl, ethyl, n-propyl, fluoromethyl, chloromethyl, and the like. Methyl group is preferable.

Examples of a lower alkenyl group is vinyl, allyl, propenyl, and the like. A preferable alkenyl group is vinyl group.

A 3-6 membered ring which is formed by $R_2$ and $R_3$ together with the adjacent carbon atom is, for example, cyclopropane ring, cyclobutane ring, or the like.

(d) $R_4$

A halogen atom may be fluorine, chlorine, bromine, or iodine atom. Fluorine and chlorine are preferable halogen atoms.

In the definition of $R_5O$-, $R_5$ represents a lower alkyl group which may have a substituent, a cyclo-lower alkyl group which may have a substituent, an aryl group which may have a substituent, a 5 or 6 membered heterocyclic group which may have a substituent, or an alkyl- or arylsulfonyl group which may have a substituent. Examples of a lower alkyl group include methyl, ethyl, and the like. A cyclo-lower alkyl group is, for example, cyclopentyl group, cyclohexyl group, or the like. A typical aryl group is phenyl group. Examples of a 5 or 6 membered heterocyclic group is thiazolyl group, oxazolyl group, and the like. An alkylsulfonyl group is exemplified by methanesulfonyl group, and an arylsulfonyl group by benzenesulfonyl group. These groups may have substituents such as a halogen atom, a hydroxyl group, a lower alkoxy group (e.g. methoxy, ethoxy, etc.), a lower alkyl group (e.g. methyl, ethyl, etc.), acyl group (e.g. acetyl, propionyl, etc.), amino group which may have substituents (e.g. methylamino, diethylamino, etc.), carboxy group, nitro group, cyano group, or the like.

In the definition of $R_6$-S, $R_6$ represents a lower alkyl group which may have a substituent, a cyclo-lower alkyl group which may have a substituent, an aryl group which may have a substituent, or a 5 or 6 membered heterocyclic group which may have a substituent. Examples of a lower alkyl group include methyl, ethyl, and the like. A cyclo-lower alkyl group is, for example, cyclopentyl group, cyclohexyl group, or the like. A typical aryl group is phenyl group. Examples of a 5 or 6 membered heterocyclic group is thiazolyl group, oxazolyl group, and the like. These groups may have substituents such as a halogen atom, a hydroxyl group, a lower alkoxy group (e.g. methoxy, ethoxy, etc.), lower alkyl group (e.g. methyl, ethyl, etc.), acyl group (e.g. acetyl, propionyl, etc.), amino group which may have substituents (e.g. methylamino, diethylamino, etc.), carboxy group, nitro group, cyano group, or the like.

In the definition of $R_7R_8N$-, $R_7$ and $R_8$ individually represent a lower alkyl group, a lower alkenyl group, a cyclo-lower alkyl group, a lower aralkyl group, an aryl group, or a 5 or 6 membered heterocyclic group, all of which may have a substituent. Examples of a lower alkyl group include methyl, ethyl, n-propyl and the

like. A lower alkenyl group includes allyl group and the like. A cyclo-lower alkyl group is, for example, cyclopentyl group, cyclohexyl group, or the like. A lower aralkyl group may be benzyl or phenetyl group. A typical aryl group is phenyl group. Examples of a 5 or 6 membered heterocyclic group include pyridyl group, imidazolyl group, thiazolyl group, oxazolyl group, and the like. These groups may have substituents such as a halogen atom, a hydroxyl group, lower alkoxy group (e.g. methoxy, ethoxy, etc.), lower acyloxy group (acetoxy, propyoxy, etc.), acyl group (e.g. acetyl, benzoyl, etc.), carboxy group, a lower alkoxycarbonyl group which may have a substituent (e.g. methoxycarbonyl, ethoxycarbonyl, etc.), aminocarbonyl group which may have a substituent (e.g. methylaminocarbonyl, dimethylaminocarbonyl, etc.), amino group which may have a substituent (e.g. amino, methylamino, ethylamino, dimethylamino, diethylamino, pyrrolidinyl, piperidinyl, benzylamino, etc.), acylamino group which may have a substituent (e.g. N-acetylmethylamino, N-acetylethylamino, etc.), aminocarbonyl group which may have a substituent (e.g. methylaminocarbonyl, ethylaminocarbonyl, etc.), cyano group, alkoxycarbonylamino group which may have a substituent (e.g. methoxycarbonylamino, etc.), or the like.

When $R_7$ and $R_8$ form a cyclic amino group together with the adjacent nitrogen atom, given as examples of such a cyclic amino group are azetidinyl, pyrrolidinyl, piperidinyl, piperazinyl, homopiperazinyl, morpholino, thiomorpholino, isooxazolidinyl, imidazolinyl, imidazolyl, pyrazolyl, pyrazolidinyl, thiazolidinyl, thiazolyl, isothiazolyl, pyrrolinyl, 4-oxopiperidinyl, 2-oxo-imdazolidinyl, 2-oxo-oxazolidinyl, and the like.

These cyclic amino groups may have substituents on the carbon atom. Such substituents include halogen atoms, hydroxyl group, alkoxy group (e.g. a lower alkoxy group such as methoxy, ethoxy, etc.), acyloxy group (e.g. acetoxy, benzoyloxy, etc.), carboxy group, alkoxycarbonyl group (e.g. ethoxycarbonyl, benzyloxycarbonyl, etc.), aminocarbonyl group which may have a substituent (e.g. methylaminocarbonyl, dimethylaminocarbonyl, etc.), alkylthio group (e.g. methylthio, ethylthio, etc.), amino group which may have a substituent (e.g. amino, methylamino, ethylamino, n-propylamino, dimethylamino, ethylmethylamino, diethylamino, benzylmethylamino, pyrrolidinyl, piperidinyl, cyclopropylamino, hydroxyethylamino, etc.), acylamino group which may have a substituent (e.g. N-acetylmethylamino, N-acetylethylamino, N-benzoylethylamino, N-acetylamino, N-benzoylamino, etc.), alkoxycarbonylamino group which may have a substituent (e.g. N-methoxycarbonylmethylamino, N-ethoxycarbonylethylamino, N-t-butoxycarbonylmethylamino, N-benzyloxycarbonylmethylamino, N-ethoxycarbonylmethylamino, N-t-butoxycarbonylethylamino, N-benzyloxycarbonylethylamino, N-t-butoxycarbonylamino, N-benzyloxycarbonylamino, etc.), lower alkyl group which may have a substituent (e.g. methyl, ethyl, n-propyl, isopropyl, n-butyl, t-butyl, fluoromethyl, chloromethyl, bromomethyl, iodomethyl, 1-chloroethyl, hydroxymethyl, 1-hydroxyethyl, 2-hydroxyethyl, 1-hydroxy-n-propyl, methoxymethyl, ethoxymethyl, benzyloxymethyl, carboxymethyl, methoxycarbonylmethyl, ethoxycarbonylmethyl, t-butoxycarbonylmethyl, benzyloxycarbonylmethyl, cyanomethyl, aminocarbonylmethyl, methylaminocarbonylmethyl, dimethylaminocarbonylmethyl, aminomethyl, methylaminomethyl, dimethylaminomethyl, ethylaminomethyl, diethylaminomethyl, n-propylaminomethyl, bi-(n-propyl)aminomethyl, isopropylaminomethyl, n-butylaminomethyl, 1-aminoethyl, 1-methylaminoethyl, 1-dimethylaminoethyl, 2-aminoethyl, 2-methylaminoethyl, 2-dimethylaminoethyl, 2-ethylaminoethyl, 2-diethylaminoethyl, 3-aminopropyl, 3-dimethylaminopropyl, pyrrolidinylmethyl, benzylaminomethyl, benzylmethylaminomethyl, N-acetylaminomethyl, N-acetylmethylaminomethyl, N-acetylethylaminomethyl, N-benzoylaminomethyl, 1-(N-acetylamino)ethyl, 1-(N-acetylmethylamino)ethyl, 1-(N-benzoylamino)ethyl, N-methoxycarbonylaminomethyl, N-ethoxycarbonylaminomethyl, N-ethoxycarbonylmethylaminomethyl, N-t-butoxycarbonylaminomethyl, N-t-butoxycarbonylmethylaminomethyl, N-t-butoxycarbonylethylaminomethyl, 1-(N-t-butoxycarbonylamino)ethyl, 1-(N-t-butoxycarbonylmethylamino)ethyl, 2-(N-t-butoxycarbonylamino)-ethyl, N-benzyloxycarbonylaminomethyl, N-benzyloxycarbonylmethylaminomethyl, N-benzyloxycarbonylethylaminomethyl, 2-hydroxyethylaminomethyl, etc.), lower alkenyl group which may have a substituent (e.g. allyl, etc.), aralkyl group which may have a substituent (e.g. benzyl, phenetyl, etc.), aryl group which may have a substituent (e.g. phenyl, etc.).

Following groups are given as examples of groups containing $-NR_9-$ in an amino group and the amino group is bi-cyclic.

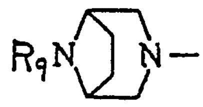

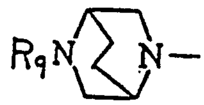

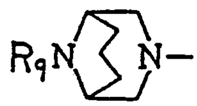

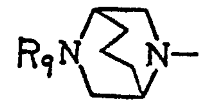

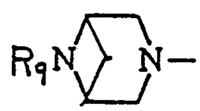

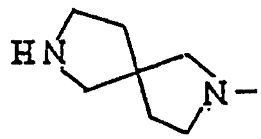

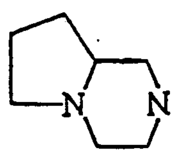

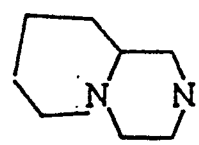

In the case where $R_7R_8N$- forms a cyclic amino group containing $-NR_9-$ in the ring, preferable examples of $R_9$ group include, as lower alkyl groups which may have a substituent, methyl, ethyl, n-propyl, n-butyl, sec-butyl, t-butyl, hydroxyethyl, methoxymethyl, ethoxymethyl, 1-methoxyethyl, 2-methoxyethyl, 2-acetoxyethyl, 3-oxoybutyl, acetonyl, and the like; as lower alkenyl groups which may have a substituent, allyl and the like; as cyclo-lower alkyl group which may have a substituent, cyclopentyl, cyclohexyl, and the like; as lower aralkyl groups which may have a substituent, benzyl, p-methoxybenzyl, and the like; as aryl group, phenyl and the like; as acyl groups which may have a substituent, formyl, acetyl, propionyl, benzoyl, and the like; as lower alkoxycarbonyl groups which may have a substituent, methoxycarbonyl, ethoxycarbonyl, t-butoxycarbonyl, and the like; as aralkyloxycarbonyl groups which may have a substituent, benzyloxycarbonyl and the like; as alkylsulfonyl groups which may have a substituent, methylsulfonyl and the like; arylsulfonyl groups which may have a substituent, benzenesulfonyl, p-toluenesulfonyl, and the like.

In the case where $R_7R_8N$- is a cyclic amino group containing a substituent on the carbon atom thereof or the nitrogen atom of $-R_9N$-, preferable examples of $R_7R_8N$- having such a substituent include 3-hydroxyazetidinyl, 3-aminoazetidinyl, 3-(N-t-butoxycarbonylamino)azetidinyl, 3-acetylaminoazetidinyl, 3-methylaminoazetidinyl, 3-dimethylaminoazetidinyl, 3-hydroxypyrrolidinyl, 3,4-dihydroxypyrrolidinyl, 3-methoxypyrrolidinyl, 3-methylpyrrolidinyl, 3-aminopyrrolidinyl, 3-methylaminopyrrolidinyl, 3-dimethylaminopyrrolidinyl, 3-ethylaminopyrrolidinyl, 3-diethylaminopyrrolidinyl, 3-aminomethylpyrrolidinyl, 3-methylaminomethylpyrrolidinyl, 3-dimethylaminomethylpyrrolidinyl, 3-ethylaminopyrrolidinyl, 3-diethylaminopyrrolidinyl, 3-(1-aminoethyl)pyrrolidinyl, 3-(2-aminoethyl)pyrrolidinyl, 3-(1-methylaminoethyl)-pyrrolidinyl, 3-methyl-3-aminopyrrolidinyl, 3-methyl-4-aminopyrrolidinyl, 3-methyl-4-methylaminopyrrolidinyl, 3-methyl-4-dimethylaminopyrrolidinyl, 2-methyl-3-aminopyrrolidinyl, 2-methyl-3-dimethylaminopyrrolidinyl, 3,4-diaminopyrrolidinyl, 3-amino-4-dimethylamino pyrrolidinyl, 3-hydroxy-4-aminopyrrolidinyl, 3-hydroxy-4-dimethylaminopyrrolidinyl, 4-hydroxypiperidinyl, 3-pyrrolidinylpyrrolidinyl, 4-aminopiperidinyl, 4-dimethylaminopiperidinyl, 4-pyrrolidinylpiperidinyl, 3-methylpiperazinyl, 4-methylpiperazinyl, 3,5-dimethylpiperazinyl, 3,4,5-trimethylpiperazinyl, 4-ethylpiperazinyl, 4-benzylpiperazinyl, 4-formylpiperazinyl, 4-acetylpiperazinyl, 4-benzoylpiperazinyl, 4-ethoxycarbonylpiperazinyl, 4-t-butoxycarbonylpiperazinyl, 4-benzyloxycarbonylpiperazinyl, 4-methylsulfonylpiperazinyl, 3-methyl-4-formylpiperazinyl, 3-methyl-4-acetylpiperazinyl, 3-methyl-4-t-butoxycarbonylpiperazinyl, 4-hydroxyethylpiperazinyl, 4-aminoethylpiperazinyl, 4-cyanomethylpiperazinyl, 4-cyanoethylpiperazinyl, 2-hydroxymethylmorpholino, 2-aminomethylmorpholino, 2-methylaminomethylmorpholino, 2-dimethylaminomethylmorpholino, 4-methylhomopiperazinyl, 2-methylimidazolyl, 2-methylimidazolidinyl, 3-methylpyrazolyl, 2-methylthiazolidinyl, and the like.

The compound of this invention may form a pharmacologically acceptable acid or base addition salt.

Examples of acid addition salts are, for example, (a) a salt of inorganic acid such as hydrochloric acid, sulfuric acid, or the like, (b) a salt of organic carboxylic acid such as formic acid, citric acid, trichloroacetic acid, trifluoroacetic acid, or the like, and (c) a salt of sulfonic acid such as methanesulfonic acid, benzenesulfonic acid, p-toluenesulfonic acid, mesitylenesulfonic acid, naphthalenesulfonic acid, or the like. Examples of base addition salts include (a) a salt of alkali metal such as sodium, potassium, or the like, (b) a salt of alkaline earth metal such as calcium, magnesium, or the like, (c) an ammonium salt, and (d) a salt of nitrogen-containing organic base such as trimethylamine, triethylamine, tributylamine, pyridine, N,N-dimethylaniline, N-methylpiperidine, N-methylmorpholine, diethylamine, dicyclohexylamine, procaine, dibenzylamine, N-benzyl-$\beta$-phenetylamine, 1-ephenamine, N,N′-dibenzylethylenediamine, or the like.

The compounds of this invention can be present in any molecular form containing or not containing a solvent combined therein. In other words, they are capable of taking any crystalline forms containing or not containing molecules of a solvent including water. Accordingly, the present invention encompasses all of crystal forms and hydrates of such compounds.

In the preparation of novel pyrido[3,2,1-ij]-[3,1]benzoxazine compounds of formula (I) of this invention, any appropriate methods can be used in forming chemical bonds in the compounds or in forming or introducing various substituents. Examples of preferable methods are given below.

[Process 1]

Among the compounds represented by the formula (I), those having a hydrogen atom or a lower alkyl group for $R_1$ and a hydrogen or halogen atom for $R_4$ can be prepared by a process shown by the following reaction formula (I),

Reaction Formula (I)

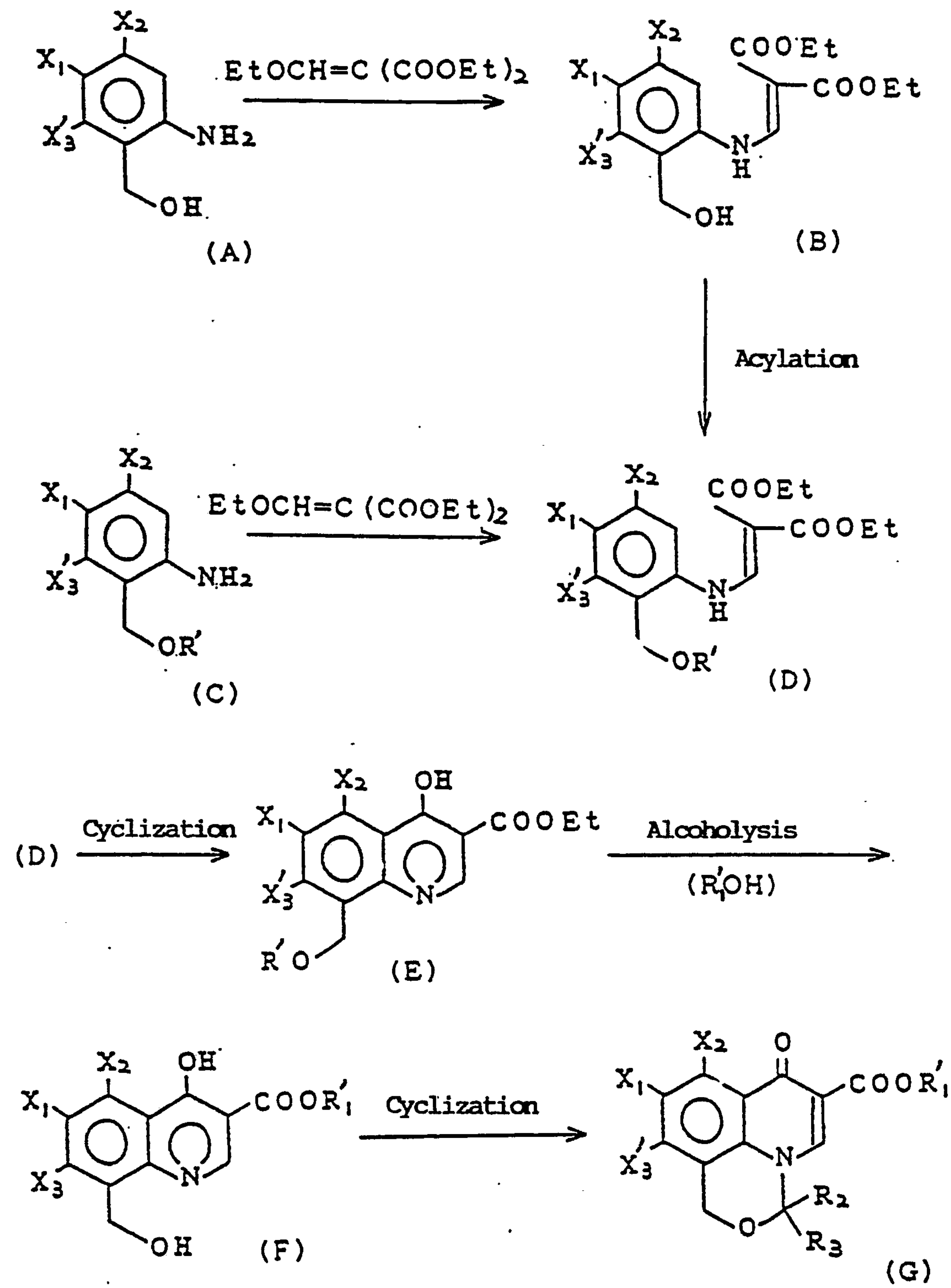

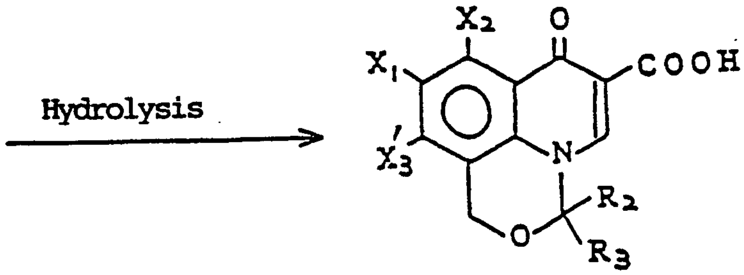


(H)

wherein $X_1$, $X_2$, $R_2$, and $R_3$ have the same meanings as previously defined, $X'_3$ represents a hydrogen or halogen atom, $R'$ is an acyl group, and $R'_1$ is a lower alkyl group.

A starting material of the process is o-hydroxymethylaniline [Compound (A)] or o-(acyloxymethyl)aniline [Compound (C)]. An acyl group ($R'$) in Compound (C) may be any group which is commonly used as a hydroxyl protective group. Examples may be acetyl, propionyl, or benzoyl group.

Compound (B) or Compound (D) is produced by reacting Compound (A) or Compound (C) with diethyl ethoxymethylene malonate. The reaction is carried out usually without using a solvent at a temperature of 80-150 °C, although it is possible to carry out the reaction in the presence of a solvent having no active hydrogen such as toluene, xylene, or the like.

Compound (B) can be converted into Compound (D) by acylation. Any acylation reaction commonly used for hydroxyl group acylation can be used for the conversion. For example, an acylchloride corresponding to $R'$ can be reacted with Compound (B) in a solvent having no active hydrogen such as dichloromethane, ether, or acetone at a temperature of 0 °C to room temperature, in the presence or absence of a base such as pyridine, triethylamine, sodium carbonate, potassium carbonate, or the like.

Compound (E) is produced by a cyclization reaction of Compound (D). The reaction can be carried out by various methods. One example is a method of heating Compound (D) at 180-270 °C in the presence of an inert solvent such as diphenylether, dibenzylether, or the like. In another method Compound (D) is heated at 100-160 °C in polyphosphoric acid.

Compound (E) is converted into Compound (F) by alcoholysis. The alcoholysis reaction is carried out at a temperature of room temperature to 100 °C using as a solvent an alcohol having $R'_1$ group with which the carboxyl group of Compound (F) or Compound (G) is to be substituted, optionally in combination with other solvent such as benzene, toluene, dimethylformamide, dimethylsulfoxide, or N-methylpyrrolidone, and using a catalytic amount of sodium hydroxide, potassium hydroxide, sodium methylate, sodium ethylate, or a base such as metal alcoholate having a group $R'_1$.

Cyclization of Compound (F) into Compound (G) can be carried out by reacting Compound (F) with a ketone or aldehyde having desired $R_2$ and $R_3$, which is represented by the formula $R_2\underset{\overset{\|}{O}}{C}R_3$, or a ketal or acetal thereof. The reaction is carried out at a temperature of room temperature to 150 °C in the presence or absence of an inert solvent such as benzene, toluene, dimethylformamide, dimethylsulfoxide, or N-methyl-pyrrolidone, and using a catalytic amount of organic or inorganic acid such as p-toluenesulfonic acid, benzenesulfonic acid, trifluoroacetic acid, sulfuric acid, hydrochloric acid, perhydrochloric acid, or the like.

Any commonly known method can be used for producing Compound (H) by hydrolysis of Compound (G). For example, a method is used in which Compound (G) is treated at a temperature of room temperature to 100 °C in an alkaline aqueous solution, such as an aqueous solution of sodium hydroxide, potassium hydroxide, sodium hydrogen carbonate, or the like, either using or without using a solvent which can dissolve into water, such as ethanol, methanol, tetrahydrofuran, dimethylformamide, or the like.

[Process 2]

Among the compounds represented by the formula (I), those having a hydrogen atom or a lower alkyl group for $R_1$ and $R_6S$- or $R_7R_8N$- for $R_4$ can be prepared by a process shown by the following reaction formula (II),

Reaction Formula (II)

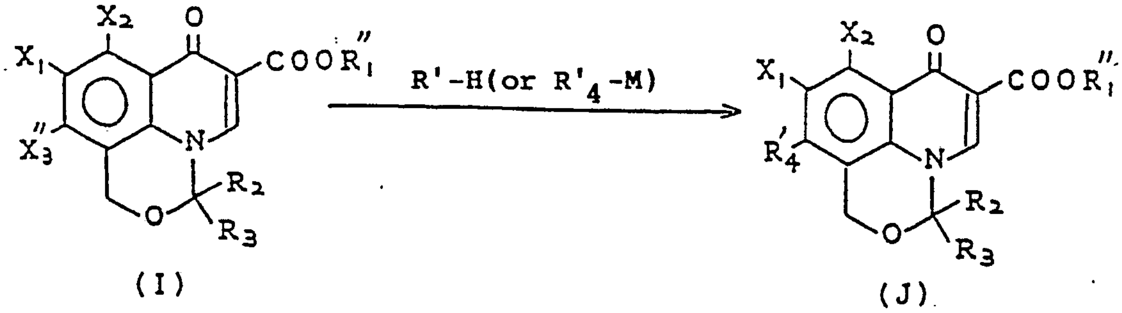

wherein $X_1$, $X_2$, $R_2$, and $R_3$, have the same meanings as previously defined for formula (I), $X''_3$ represents a fluorine or chlorine atom, $R''_1$ represents a hydrogen atom or a lower alkyl group, $R'_4$ has the meaning as previously defined in connection with $R_6S$ and $R_7R_8N$, and M is an alkali metal.

This process comprises reacting Compound (I) which is produced in Process 1 with $R'_4$-H or its alkali metal compound $R'_4$-M.

The reaction is desirably carried out in the presence of an inert solvent such as acetonitrile, dimethylformamide, dimethylsulfoxide, N-methylpyrrolidone, or the like, or a mixture of these solvents, at a temperature of 50-200° C, preferably of 80-150° C. It is desirable to use a base such as triethylamine, lutidine, 1,4-diazabicyclo[5,4,0]-undeca-7-ene, 1,5-diazabicyclo[4,3,0]nona-5-ene, an alakli metal carbonate, or the like in order to neutralize hydrogen fluoride or hydrogen chloride which is produced in the reaction. Another method of neutralizing hydrogen fluoride or hydrogen chloride produced in the reaction is to use an excess amount of an alkali metal compound or $R_7R_8NH$.

In this reaction, the product may be obtained as a salt. If necessary, the salt can be converted into a free acid by treating with an acid such as acetic acid, trifluoroacetic acid, hydrochloric acid, or the like, or with a base such as sodium carbonate, potassium carbonate, sodium hydroxide, potassium hydroxide, or the like.

In case where $R_6$, $R_7$, or $R_8$ in the group $R'_4$ has a hydroxyl, mercapto, carboxyl, or amino group substituent, an yield of Compound (J) in the above reaction may be extremely decreased. In such a case, a better yield can be attained if $R_6$, $R_7$, or $R_8$ can be subjected to the reaction of Process 2 after the hydroxyl, mercapto, carboxyl, or amino group substituent is modified with a suitable protective group. The protective group may be removed after the reaction. Any conventionally known protective groups can be used. Examples include, for protecting hydroxyl group, acyl, alkoxycarbonyl, or banzyl group; for protecting mercapto group, acyl or ethylthio group; for protecting carboxyl group, banzyl group; for protecting amino group, acyl, alkoxycarbonyl, or banzyl group. These protective groups can be removed after the reaction by a commonly known method such as acid hydrolysis, base hydrolysis, or catalytic hydrogenation.

[Process 3]

Among the compounds represented by the formula (I), those having a hydrogen atom or a lower alkyl group for $R_1$ and a hydroxyl group or $R_5O$- for $R_4$ can be prepared by a process shown in the following reaction formula (III),

**Reaction Formula (III)**

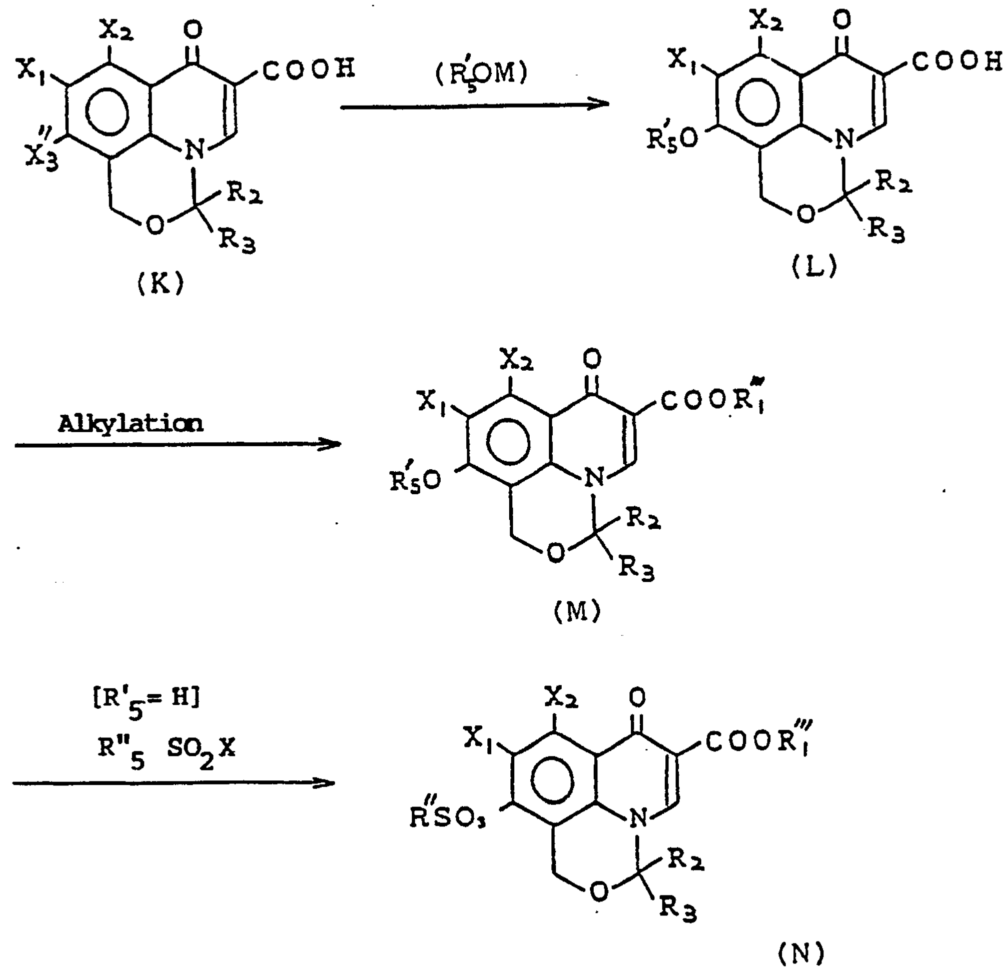


wherein $X_1$, $X_2$, $R_2$ and $R_3$ have the same meanings as previously defined for formula (I), $X'_3$ represents a fluorine or chlorine atom, $R'_5$ represents a hydrogen atom or the same group as defined for $R_5$ of formula (I) excluding alkylsulfonyl and arylsulfonyl groups, $R'''_1$ represents the same lower alkyl group as defined for $R_1$ of formula (I), $R''_5$ represents alkyl or aryl group, M is an alkali metal, and X is a halogen atom.

In the step for producing Compound (L) from Compound (K), a compound represented by $R'_5OH$ or its alkali metal compound is reacted with Compound (K). The reaction is carried out under the same conditions as described for Process 2.

The step for producing Compound (M) from Compound (L) can be carried out by reacting Compound (L) with a lower alkyl halide, preferably with a lower alkyl iodide or bromide, having an $R'_1$ group. It is desirable the reaction be carried out in the presence of a base such as triethylamine, trimethylamine, sodium hydroxide, potassium hydroxide, sodium carbonate, potassium carbonate, or the like, and an inert solvent such as dimethylformamide, dimethylsulfoxide, N-methylpyrrolidone, or the like. A preferable temperature range is from room temperature to 100 °C. The same problem of a reduced yield on account of the substituent on $R'_5$ discussed in connection with Process 2 could take place in this reaction. The problem can be solved in the same manner as in Process 2.

Compound (N) can be prepared by reacting Compound (M) having hydrogen atom for $R'_5$ with an alkylsulfonyl halide or arylsulfonyl halide having a formula of $R''_5SO_2X$. It is desirable to carry out this reaction in the presence of a base such as triethylamine, pyridine, N,N-dimethylaniline, potassium carbonate, sodium carbonate, or the like, and an inert solvent such as benzene, acetonitrile, dimethylformamide, dimethylsulfoxide, N-methylpyrrolidone, or the like. A preferable reaction temperature range is from room temperature to 80 °C.

[Process 4]

Among the compounds represented by the formula (I), those having a carboxyl protective group for $R_1$ can be prepared by a process shown, for example, by the following reaction formula (IV),

**Reaction Formula (IV)**

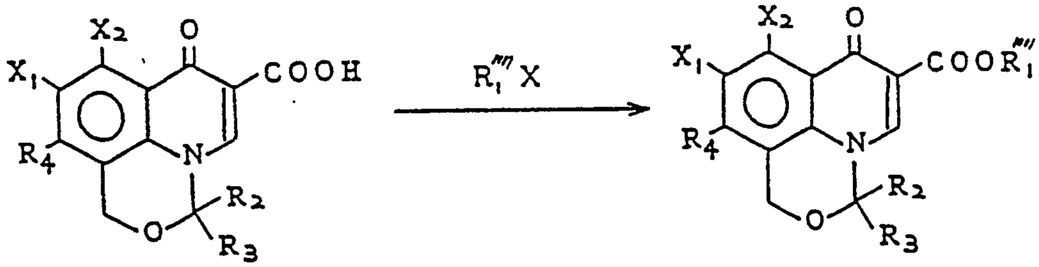


(O) (P)

wherein $X_1$, $X_2$, $R_2$, $R_3$, and $R_4$ have the same meanings as previously defined for formula (I), $R''''_1$ represents the same carboxy protective group as defined for $R_1$, and X is a halogen atom.

Compound (P) is prepared by reacting Compound (O) with a halide $R''''_1X$. The reaction is carried out using the same conditions as those used for the conversion of Compound (L) into Compound (M) as discussed in Process 3.

Processes 1-4 discussed above are examples for the preparation of novel pyrido[3,2,1-ij][3,1]-benzoxazine compounds of formula (I) of this invention. Methods for the production of the compounds of formula (I) are by no means limited to these Processes 1-4. Especially, substituents in the groups represented by $R_4$ can be interchangeable by a variety of known methods. An example of such interchange of substituents was discussed in Process 2 in connection with protective group attachment and detachment. Other examples of such substituent interchange are the conversion of a hydroxyl group into halogen atom, acylation of hydroxyl, mercapto, or primary- or secondary amino group, conversion of halogen atom into amino or cyano group, removal of alkoxycarbonyl, benzyl, or acyl group, alkylation of hydroxyl, mercapto, carboxyl, or primary- or secondary amino group, sulfonylation of primary- or secondary amino group, conversion of alkenyl group into alkyl group, cyclization of two or more substituents. Efficient preparation of pyrido[3,2,1-ij][3,1]benzoxazine compounds of formula (I) of this invention is possible by siutable combinations of these reactions.

Compounds (A) or (C) which are the starting materials of Process 1 can be prepared according to the following reaction.

**Reaction Formula (V)**

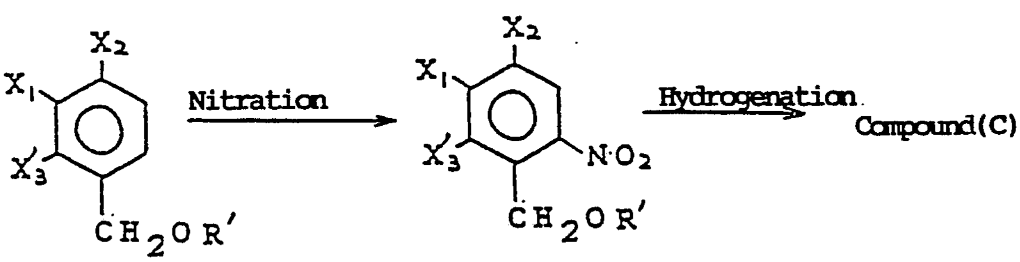

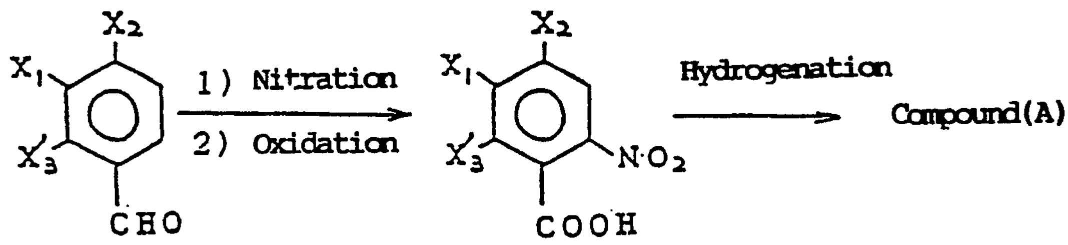


wherein all symbol designation of various substituents have the same meanings as defined in formula (I).

Other features of the invention will become apparent in the course of the following description of the exemplary embodiments which are given for illustration of the invention and are not intended to be limiting thereof.

## Example

### Reference Example 1

Synthesis of 2-acetoxymethyl-3,4-difluoroaniline

To 50 ml of dichloromethane were added 14.4 g of 2,3-difluorobenzyl alcohol and 8.7 g of pyridine. The mixture was ice-cooled and a solution of 8.8 g of acetylchloride in 20 ml of dichloromethane was added dropwise while stirring. After stirring for 1 hour at room temperature, water was added to effect phase separation. The dichloromethane layer was washed with 2% aqueous solution of sodium hydrogen carbonate, dried over anhydrous sodium sulfate, and concentrated under reduced pressure to produce 2,3-difluoroacetoxymethylbenzene as a colorless oil.

The whole amount of 2,3-difluoroacetoxymethylbenzene was added dropwise to 70 ml of ice-cooled fuming nitric acid while stirring. After stirring for 30 minutes under ice-cooling, the mixture was added to 500 ml of ice-cold water and extracted with 150 ml of dichloromethane. The dichloromethane layer was washed with 2% aqueous solution of sodium hydrogen carbonate, dried over anhydrous sodium sulfate, and concentrated under reduced pressure to produce a colorless oil. $^1$H-NMR analysis confirmed this product to be an approximately 3:2 mixture of 2-acetoxymethyl-3,4-difluoronitrobenzene [$^1$H-NMR, δ $CDCl_3$; 2.70(3H, s), 5.47(2H, s), 7.35(1H, dt, J=9Hz, 8Hz), 7.86(1H, ddd, J=9Hz, 4Hz, 2Hz)], and 3-acetoxymethyl-4,5-difluoronitrobenzene [$^1$H-NMR, δ $CDCl_3$; 5.24(3H, s), 2.18(3H, s), 8.03-8.17(2H, m)].

The whole amount of the above mixture was mixed with 220 ml of methanol and stirred overnight in a hydrogen atmosphere in the presence of 2 g of 5% palladium-carbon. After removal of the catalyst by filtration, the filtrate was concentrated under reduced pressure and the residue was subjected to silica gel column chromatography to obtain 10 g of 2-acetoxymethyl-3,4-difluoroaniline as a colorless oil [$^1$H-NMR, 6 $CDCl_3$; 2.10(3H, s), 5.18(2H, s), 6.37(1H, ddd, J=9Hz, 4Hz, 2Hz), 6.96(1H, dt, J=9Hz, 8Hz)

### Reference Example 2

Synthesis of 3,4-dichloro-2-hydroxymethylaniline

To a mixture of each 200 ml of concentrated nitric acid and concentrated sulfuric acid was added 105 g of 2,3- dichlorobenzaldehyde, followed by stirring at 90-100 °C for 18 hours. After cooling to the room temperature, the resultant reaction mixture was added to 5 l of ice-cold water to collect the deposited substances by filtration. The deposit was dissolved into 1 l of chloroform, washed with water, dried over anhydrous sodium sulfate, and concentrated under reduced pressure to produce a solid residue. The residue was recrystallized in methanol to produce 50.5 g of 2,3-dichloro-6-nitrobenzoic acid. The filtrate was concentrated under reduced pressure and a solid residue was recrystallized in methanol to produce 25 g of

2,3-dichloro-6-nitrobenzoic acid, thus obtaining 75.5 g of 2,3-dichloro-6-nitrobenzoic acid (1/2 methanol adduct, mp: 118-120 °C) in total.

16 g of the above 2,3-dichloro-6-nitrobenzoic acid was mixed with 150 ml of methanol and stirred overnight in a hydrogen atmosphere in the presence of 1 g of 5% palladium-carbon. After removal of the catalyst by filtration, the filtrate was concentrated under reduced pressure. Deposited colorless needle-like crystals were collected by filtration, washed with chloroform to produce 10.5 g of 6-amino-2,3-dichlorobenzoic acid (mp: 163-164 °C).

4.1 g of 6-amino-2,3-dichlorobenzoic acid was added, a bit at a time, to a dispersion of 1.7 g of lithium aluminum hydride in 200 ml of tetrahydrofuran, and the mixture was stirred at the room temperature for 2 hours. Small amounts of methanol and then water were added to the resulting reaction mixture to decompose surplus hydride. The deposit thus produced was removed by filtration. 200 ml of water and 200 ml of chloroform were added to the filtrate to separate layers. Colorless needle-like crystals deposited from the chloroform layer was collected by filtration and washed with diisopropyl ether to produce 2.4 g of 3,4-dichloro-2-hydroxymethylaniline (mp: 136-137.5 °C).

Reference Example 3

Synthesis of diethyl N-(2-acetoxymethyl-3,4-difluorophenyl)aminomethylene malonate

A mixture of 2.36 g of 2-acetoxymethyl-3,4-difluoroaniline and 2.45 g of diethyl ethoxymethylene malonate was heated at 120 °C with stirring for 3 hours. The resultant reaction mixture was cooled to the room temperature to collect deposited colorless crystals, which were washed with diisopropyl ether to obtain 2.93 g of diethyl N-(2-acetoxymethyl-3,4-difluorophenyl)aminomethylene malonate (mp: 108-109.5 °C).

Reference Example 4

Synthesis of diethyl N-(3,4-dichloro-2-hydroxymethylphenyl)aminomethylene malonate

A mixture of 1.92 g of 3,4-dichloror-2-hydroxymethylaniline and 2.36 g of diethyl ethoxymethylene malonate was heated at 120 °C with stirring for 1 hour. The resultant reaction mixture was cooled to the room temperature to collect deposited colorless needle-like crystals, which were washed with diisopropyl ether to obtain 3.35 g of diethyl N-(3,4-dichloro-2-hydroxymethylphenyl)aminomethylene malonate (mp: 140-142 °C).

Reference Example 5

Synthesis of diethyl N-(2-acetoxymethyl-3,4-dichlorophenyl)aminomethylene malonate

1.6 g of diethyl N-(3,4-dichloro-2-hydroxymethylphenyl)aminomethylene malonate and 2 ml of triethylamine were dissolved in 20 ml of dichloromethane. To the mixture was dropwise added a solution of 0.6 g of acetylchloride in 6 ml of dichloromethane at room temperature while stirring. After the addition, the mixture was washed with water, dried over anhydrous sodium sulfate, and concentrated under reduced pressure. Deposited colorless crystals were collected by filtration and washed with diisopropyl ether to obtain 1.66 g of diethyl N-(2-acetoxymethyl-3,4-dichlorophenyl)aminomethylene malonate (mp: 148-150 °C).

Reference Example 6

Synthesis of ethyl 8-acetoxymethyl-6,7-difluoro-4-hydroxyquinoline-3-carboxylate

9.6 g of diethyl N-(2-acetoxymethyl-3,4-difluorophenyl)aminomethylene malonate was added to 100 ml of diphenyl ether and heated under refluxing for 20 minutes. After cooling to the room temperature, the deposited substances were collected by by filtration, and washed with diisopropyl ether to produce 6.67 g of pale brown solid ethyl 8-acetoxymethyl-6,7-difluoro-4-hydroxyquinoline-3-carboxylate. Colorless crystals of this compound were obtained by recrystallization from N,N-dimethylformamide [mp: 246-250° C (decomposed); $^1$H-NMR, δ $CDCl_3$; 1.48(3H, t, J=7Hz), 2.09(3H, s), 4.50(2H, q, J=7Hz), 5.78(2H, s), 8.09(1H, dd, J=10Hz, 8Hz), 9.17(1H, s)].

Reference Example 7

Synthesis of ethyl 8-acetoxymethyl-6,7-dichloro-4-hydroxyquinoline-3-carboxylate

400 mg of ethyl 8-acetoxymethyl-6,7-dichloro-4-hydroxyquinoline-3-carboxylate was prepared in the same manner as in Reference Example 6, using 780 mg of diethyl N-(2-acetoxymethyl-3,4-dichlorophenyl)-aminomethylene malonate [mp: above 295° C; $^1$H-NMR, δ $CDCl_3$; 1.46(3H, t, J=7Hz), 2.09(3H, s), 4.51(2H, q, J=7Hz), 5.93(2H, s), 8.50(1H, s), 9.17(1H, s)].

Reference Example 8

Synthesis of ethyl 6,7-difluoro-8-hydroxymethyl-4-hydroxyquinoline-3-carboxylate

3.0 g of ethyl 8-acetoxymethyl-6,7-difluoro-4-hydroxyquinoline-3-carboxylate and 0.5 g of sodium methoxide were added to 150 ml of ethanol and heated under refluxing. After cooling, half the amount of ethanol was evaporated under reduced pressure. The deposited substances were collected by filtration, and washed with ethanol to produce 2.45 g of ethyl 6,7-difluoro-8-hydroxymethyl-4-hydroxy quinoline-3-carboxylate as a pale brown solid. Colorless crystals of this compound were obtained by recrystallization from N,N-dimethylformamide
[mp: 242-246° C; $^1$H-NMR, δ $CDCl_3$-$CD_3OD$ (10:1); 1.42(3H, t, J=7Hz), 4.41(2H, q, J=7Hz), 5.10(2H, s), 8.08(1H, dd, J=10Hz, 8Hz), 8.70(1H, s)].

Reference Example 9

Synthesis of ethyl 6,7-dichloro-8-hydroxymethyl-4-hydroxyquinoline-3-carboxylate

In the same manner as in Reference Example 8, but using 350 mg of ethyl 8-acetoxymethyl-6,7-dichloro-4-hydroxyquinoline-3-carboxylate, 275 mg of ethyl 6,7-dichloro-8-hydroxymethyl-4-hydroxyquinoline-3-carboxylate was prepared [mp: 253-258° C; $^1$H-NMR, δ $CDCl_3$-$CD_3OD$ (10:1); 1.38(3H, t, J=7Hz), 4.30(2H, q, J=7Hz), 5.26(2H, s), 8.33(1H, s), 8.86(1H, s)].

Example 1

Synthesis of ethyl 9,10-difluoro-3-methyl-7-oxo-1H,3H,7H-pyrido[3,2,1-ij][3,1]benzoxadine-6-carboxylate

A mixture of 2.0 g of ethyl 6,7-difluoro-8-hydroxymethyl-4-hydroxyquinoline-3-carboxylate, 1.1 g of p-toluenesulfonic acid hydrate, 10 ml of acetaldehyde diethyl acetal, and 6 ml of N-methylpyrrolidone was stirred at 80° C for 1.5 hours. After cooling to the room temperature, 200 ml of chloroform was added and the mixture was washed with 5% aqueous solution of sodium hydrogen carbonate. The chloroform layer was dried with anhydrous sodium sulfate and concentrated under reduced pressure. Colorless crystals deposited was dispersed into diisopropyl ether, collected by filtration, and washed with the same solvent to obtain 1.82 g of the title compound [mp: 219-219.5° C; $^1$H-NMR, δ $CDCl_3$; 1.41(3H, t, J=7Hz), 1.87(3H, d,

J = 6Hz), 4.40(2H, q, J = 7Hz), 5.16(2H, *), 5.59(1H, q, J = 6Hz), 8.14(1H, dd, J = 10Hz, 8Hz), 8.46(1H, s)].
* designates the central value of AB pattern.

Example 2

Synthesis of ethyl 9,10-difluoro-3-ethyl-7-oxo-1H,3H,7H-pyrido[3,2,1-ij][3,1]benzoxadine-6-carboxylate

The same procedure as in Example 1 was followed, except that propionaldehyde diethyl acetal was used instead of acetaldehyde diethyl acetal to produce colorless crystals of the title compound [mp: 137-138.5° C; $^1$H-NMR, δ $CDCl_3$; 1.14(3H, t, J = 7Hz), 1.41(3H, t, J = 7Hz), 2.05-2.16(2H, m), 4.40(2H, q, J = 7Hz), 5.13(2H, *), 5.40(2H, dd, J = 7Hz, 5Hz), 8.16(1H, dd, J = 10Hz, 8Hz), 8.43(1H, s)].

Example 3

Synthesis of ethyl 9,10-dichloro-3-methyl-7-oxo-1H,3H,7H-pyrido[3,2,1-ij][3,1]benzoxadine-6-carboxylate

The same procedure as in Example 1 was followed, except that ethyl 6,7-dichloro-8-hydroxymethyl-4-hydroxyquinoline-3-carboxylate was used instead of ethyl 6,7-difluoro-8- hydroxymethyl-4-hydroxyquinoline-3-carboxylate to produce colorless crystals of the title compound [mp: 221.5-222° C; $^1$H-NMR, δ $CDCl_3$; 1.41(3H, t, J = 7Hz), 1.85(3H, d, J = 6Hz), 4.40(2H, q, J = 7Hz), 5.11(2H, *), 5.57(1H, q, J = 6Hz), 8.46(1H, s)].

Example 4

Synthesis of methyl 9,10-difluoro-3,3-dimethyl-7-oxo-1H,3H,7H-pyrido[3,2,1-ij][3,1]benzoxadine-6-carboxylate

The same procedure as in Example 1 was followed, except that 2,2-dimethoxypropane was used instead of acetaldehyde diethyl acetal to produce colorless solid of the title compound [$^1$H-NMR, δ $CDCl_3$; 1.85(s), 3.94(s), 5.13(s), 8.19(dd, J = 10Hz, 8Hz), 8.62(s)] as a mixture of ethyl ester [$^1$H-NMR, δ $CDCl_3$; 1.42-(t, J = 7Hz), 1.85(s), 4.41(q, J = 7Hz), 5.13(s), 8.19(dd, J = 10Hz, 8Hz), 8.60(s)].

Example 5

Synthesis of ethyl 9,10-difluoro-7-oxo-[cyclohexanespiro]-3-1H,3H,7H-pyrido[3,2,1-ij][3,1]benzoxadine-6-carboxylate

The same procedure as in Example 1 was followed, except that cyclohexanonedimethyl acetal was used instead of acetaldehyde diethyl acetal to produce colorless solid of the title compound [$^1$H-NMR, δ $CDCl_3$; 1.42(t, J = 7Hz), 1.70-2.04(m), 2.16-2.29(m), 4.41(q, J = 7Hz), 5.09(s), 8.18(dd, J = 10Hz, 8Hz), 8.66(s)] as a mixture of methyl ester [$^1$H-NMR, δ $CDCl_3$; 1.70-2.04(m), 2.16-2.29(m), 3.94(s), 5.09(s), 8.18(dd, J = 10Hz, 8Hz), 8.68(s)].

Example 6

Synthesis of 9,10-difluoro-3-methyl-7-oxo-1H,3H,7H-pyrido[3,2,1-ij][3,1]benzoxadine-6-carboxylic acid

620 mg of ethyl 9,10-difluoro-3-methyl-7-oxo-1H,3H,7H-pyrido[3,2,1-ij][3,1]benzoxadine-6-carboxylate was added to a solution of 230 mg of potassium hydroxide in 1.5 ml of water and 5 ml of ethanol. The

mixture was stirred at 60°C for 10 minutes. After an addition of 230 ml of acetic acid and stirring for 5 minutes at the same temperature, the mixture was allowed to cooled and the deposited substance was washed with water, ethanol, and diisopropyl ether in this order to produce colorless powder of the title compound [$^1$H-NMR, δ $CDCl_3$; 1.93(3H, t, J=6Hz), 5.23(2H, *), 5.69(1H, q, J=6Hz), 8.20(1H, t, J=9Hz), 8.77(1H, s)] containing a small amount of 10-ethoxy-9-fluoro-3-methyl-7-oxo-1H,3H,7H-pyrido[3,2,1-ij][3,1]-benzoxadine-6-carboxylic acid [$^1$H-NMR, δ $CDCl_3$; 1.44(3H, t, J=7Hz), 1.89(3H, d, J=6Hz), 4.45(2H, q, J=7Hz), 5.12(2H, *), 5.63(1H, q, J=6Hz), 8.80(1H, d, J=12Hz), 8.69(1H, s)]. Colorless crystals of this compound were obtained by recrystallization from N,N-dimethylformamide [mp: 270-273°C (decomposed)].

Example 7

Synthesis of 9,10-difluoro-3-ethyl-7-oxo-1H,3H,7H-pyrido[3,2,1-ij][3,1]benzoxadine-6-carboxylic acid

The title compound in a form of colorless powder [1H-NMR, δ $CDCl_3$; 1.17(3H, t, J=7Hz), 2.06-2.22(2H, m), 5.20(2H, *), 5.54(1H, t, J=6Hz), 8.19(1H, t, J=9Hz), 8.73(1H, s)] containing a small amount of 10-ethoxy-9-fluoro-3-ethyl-7-oxo-1H,3H,7H-pyrido[3,2,1-ij][3,1]benzoxadine-6-carboxylic acid [$^1$H-NMR, δ $CDCl_3$; 1.17(3H, t, J=7Hz), 1.44(3H, t, J=7Hz), 2.06-2.22(2H, m), 4.45(2H, q, J=7Hz), 5.08(2H, *), 5.47(1H, t, J=6Hz), 8.07(1H, d, J=12Hz), 8.66(1H, s)] as an impurity was prepared in the same manner as in Example 6 but using the compounds prepared in Example 2 as a starting material.

Example 8

Synthesis of 9,10-dichloro-3-methyl-7-oxo-1H,3H,7H-pyrido[3,2,1-ij][3,1]benzoxadine-6-carboxylic acid

The title compound in a form of colorless powder [mp: 287-292 (decomposed); $^1$H-NMR, δ $CDCl_3$; 1.92-(3H, d, J=6Hz), 5.18(2H, *), 5.67(1H, q, J=6Hz), 8.53(1H, s), 8.75(1H, s)] was prepared in the same manner as in Example 6, but using the compounds prepared in Example 3 as a starting material.

Example 9

Synthesis of 9,10-difluoro-3,3-dimethyl-7-oxo-1H,3H,7H-pyrido[3,2,1-ij][3,1]benzoxadine-6-carboxylic acid

The title compound in a form of colorless powder [$^1$H-NMR, δ $CDCl_3$; 1.90(6H, s), 5.20(2H, s), 8.22(1H, t, J=9Hz), 8.87(1H, s)] containing a small amount of 10-ethoxy-9-fluoro-3,3-dimethyl-7-oxo-1H,3H,7H-pyrido-[3,2,1-ij][3,1]benzoxadine-6-carboxylic acid [$^1$H-NMR, δ $CDCl_3$; 1.45(3H, t, J=7Hz), 1.86(6H, s), 4.47(2H, q, J=7Hz), 5.08(2H, s), 8.12(1H, α, J=12Hz), 8.81(1H, s)] as an impurity was prepared in the same manner as in Example 6 but using the compounds prepared in Example 4 as a starting material.

Example 10

Synthesis of 9-fluoro-10-(methylpiperadinyl)-3-methyl-7-oxo-1H,3H,7H-pyrido[3,2,1-ij][3,1]benzoxadine-6-carboxylic acid

To 300 mg of dimethylsulfoxide were added 100 mg of 9,10-difluoro-3-methyl-7-oxo-1H,3H,7H-pyrido-[3,2,1-ij][3,1]benzoxadine-6-carboxylic acid and 180 mg N-methylpiperadine, and the mixture was stirred for 30 minutes at 130°C. Pale yellow needle-like crystals produced by allowing the mixture at room temperature was collected by filtration and washed with ethanol and then diisopropyl ether to obtain 67 mg of the title compound [mp: 249-253°C; recrystallized from N,N-dimethylformamide: $^1$H-NMR, δ $CDCl_3$; 1.91-(3H, d, J=6Hz), 2.38(3H, s), 2.56(4H, brs), 3.22(4H, brs), 5.12(2H, *), 5.65(1H, q, J=6Hz), 8.68(1H, s)]

Examples 11-19

Derivatives of 9,10-difluoro-3-methyl-7-oxo- 1H,3H,7H-pyrido[3,2,1-ij][3,1]benzoxadine-6-carboxylic acid having a cyclic amino group in place of the fluorine atom at 10 position as shown in Table 1 were prepared in the same manner as in Example 10, except that N-methylpiperadine was replaced with various cyclic amines.

TABLE 1

| Example No. | Substituent at 10-Position | Property | Melting Point |
|---|---|---|---|
| 11 | | Colorless crystals | 286-278° C (decomposed) |
| 12 | | Colorless crystals | 242-243° C (decomposed) |
| 13 | | Colorless crystals | 275-277.5° C (decomposed) |
| 14 | | Pale brown crystals | 274-282° C (decomposed) |
| 15 | | Colorless crystals | above 295° C |
| 16 | | Pale yellow crystals | above 295° C |
| 17 | | Pale yellow crystals | 278-281° C (decomposed) |
| 18 | | Colorless crystals | 227-230° C (decomposed) |
| 19 | | Colorless powder | above 295° C |

Example 20

Synthesis of 10-(3-aminopyrrolidinyl)-9-fluoro-3-methyl-7-oxo-1H,3H,7H- pyrido[3,2,1-ij][3,1]benzoxadine-6-carboxylic acid

To 500 mg of dimethylsulfoxide were added 100 mg of 9,10-difluoro-3-methyl-7-oxo-1H,3H,7H-pyrido-[3,2,1-ij]-[3,1]benzoxadine-6-carboxylic acid, 100 mg of 3-aminopyrrolidine dihydrochloride, and 300 mg 1,8-diazabicyclo-[5,4,0]-7-undecene, and the mixture was stirred for 1 hour at 100° C. Crystals produced by allowing the mixture at room temperature was collected by filtration and washed with ethanol and then diisopropyl ether to produce 36 mg of the title compound [mp: 228-233° C (decomposed); $^{1}$H-NMR, δ $CDCl_3$-$CD_3OD$ (10:1); 1.72-1.95(1H, m), 1.92(3H, d, J=6Hz), 2.16-2.25(1H, m), 3.11-3.80(5H, m), 5.05(2H, *), 5.71(1H, q, J=6Hz), 7.94(1H, q, J=13Hz), 8.67(1H, s)]

Examples 21-27

Derivatives of 9,10-difluoro-3-methyl-7-oxo-1H,3H,7H-pyrido[3,2,1-ij][3,1]benzoxadine-6-carboxylic acid having a cyclic amino group in place of the fluorine atom at 10 position as shown in Table 2 were prepared in the same manner as in Example 20 by using suitable cyclic amine or their hydrochloride instead of aminopyrrolidine dihydrochloride.

TABLE 2

| Example No. | Substituent at 10-Position | Property | Melting Point |
|---|---|---|---|
| 21 | | Colorless crystals | 220-225 °C (decomposed) |
| 22 | | Pale red powder | 193-196 °C (decomposed) |
| 23 | | Colorless crystals | 228-231 °C (decomposed) |
| 24 | | Colorless crystals | 245-248 °C (decomposed) |
| 25 | | Pale yellow crystals | 201-211 °C |
| 26 | | Pale yellow crystals | 188-192 °C |
| 27 | | Pale yellow crystals | 255-259 °C (decomposed) |

Example 28

Synthesis of 9-fluoro-10-piperadinyl-3,3-dimethyl-7-oxo-1H,3H,7H- pyrido[3,2,1-ij][3,1]benzoxadine-6-carboxylic acid

36 mg of the title compound was prepared in the same manner as Example 10, except that 9,10-difluoro-3,3-dimethyl-7-oxo-1H,3H,7H-pyrido[3,2,1-ij][3,1]benzoxadine-6-carboxylic acid was used instead of 9,10-difluoro-3-methyl-7-oxo-1H,3H,7H-pyrido[3,2,1-ij][3,1]benzoxadine-6-carboxylic acid [mp: 249-253 °C (decomposed)].

Experimental Example

Minimum inhibitive concentrations (MIC: µg/ml) of the compounds prepared in Examples 10, 11, and 21 were measured according to the standard method of Japan Chemotherapy Association [Chemotherapy, 29, No. 1, 76-79 (1981)]. The results are shown in Table 3.

TABLE 3

| In vitro Antimicrobial Activity (MIC: µg/ml) | | | |
|---|---|---|---|
| | Tested Microorganisms | | |
| | *S. aureus* 209P | *E.coli* NIHJ-JC2 | *P.aeruginosa* |
| Compounds | (IFO 12732) | (IFO 12734) | (IFO 3445)* |
| Example 10 | 1.56 | 0.78 | 3.13 |
| Example 11 | 0.78 | 0.39 | 1.56 |
| Example 12 | 0.05 | 0.78 | 1.56 |

Obviously, numerous modifications and variations of the present invention are possible in light of the above teachings. It is therefore to be understood that within the scope of the appended claims, the invention may be practiced otherwise than as specifically described herein.

**Claims**

1. A pyridobenzoxazine derivative represented by the formula (I) or a salt thereof.

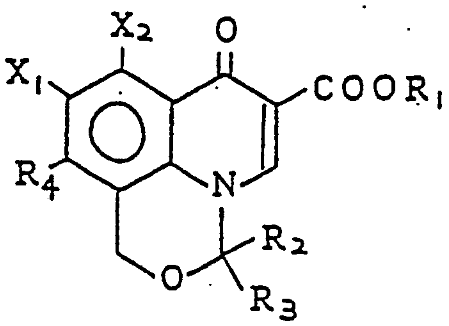

(I)

wherein $X_1$ and $X_2$ individually represent a hydrogen atom or a halogen atom; $R_1$ represents a hydrogen atom or a carboxyl protective group; $R_2$ and $R_3$ individually represent a hydrogen atom, a lower alkyl group which may be substituted by halogen atoms, or a lower alkenyl group; or $R_2$ and $R_3$ may, in combination, form a 3-6 membered ring together with the adjacent carbon atom; $R_4$ represents a hydrogen atom, a hydroxyl group, a group $R_5$-O, wherein $R_5$ represents a lower alkyl group which may have a substituent, a cyclo-lower alkyl group which may have a substituent, an aryl group which may have a substituent, a 5 or 6 membered heterocyclic group which may have a substituent, or an alkyl- or arylsulfonyl group which may have a substituent; a group $R_6S$-, wherein $R_6$ represents a lower alkyl group which may have a substituent, a cyclo-lower alkyl group which may have a substituent, an aryl group which may have a substituent, or a 5 or 6 membered heterocyclic group which may have a substituent; or a group $R_7R_8N$-, wherein $R_7$ and $R_8$ individually represent a hydrogen atom, a lower alkyl group which may have a substituent, a lower alkenyl group which may have a substituent, a cyclo-lower alkyl group which may have a substituent, a lower aralkyl group which may have a substituent, an aryl group which may have a substituent, a 5 or 6 membered heterocyclic group which may have a substituent, or $R_7$ and $R_8$ may, in combination, form a 4-7 membered cyclic amino group together with the adjacent nitrogen atom; provided (i) that the carbon atoms of the cyclic amino group may have one or more substituents selected from the group consisting of halogen atom, hydroxyl group, alkoxy group, acyloxy group, carboxyl group, alkoxycarbonyl group, aminocarbonyl group which may have a substituent, cyano group, mercapto group, alkylthio group, amino group which may have a substituent, acylamino group which may have a substituent, alkoxycarbonylamino group which may have a substituent, lower alkyl group which may have a substituent, lower alkenyl group which may have a substituent, cyclo-lower alkyl group which may have a substituent, aralkyl group which may have a substituent, and aryl group which may have a substituent, provided that when carbon atoms have two or more substituents which are lower alkyl groups which may have a substituent, the two lower alkyl groups which may have a substituent and the cyclic amino group may together form a bi-cyclic amino group; (ii) that said cyclic amino group or said bi-cyclic amino group may contain in their rings a group $-NR_9$, $-\underset{\underset{O}{\|}}{C}-$, -O-, -S-, -SO-, or $-SO_2$-; wherein $R_9$ is a hydrogen atom, a hydroxyl group, lower alkyl group which may have a substituent, lower alkenyl group which may have a substituent, cyclo-lower alkyl group which may have a substituent, aralkyl group which may have a substituent, aryl group which may have a substituent, acyl group which may have a substituent, a lower alkyloxycarbonyl group which may have a substituent, aralkyloxycarbonyl group which may have a substituent, alkylsulfonyl group which may have a substituent, or arylsulfonyl group which may have a substituent; provided that when $-NR_9$- is contained in the ring, $R_9$ is a lower alkyl group which may have a substituent, and at least one lower alkyl group which may have a substituent is present on a carbon atom or carbon atoms of the cyclic amino group, one of the lower alkyl groups and said cyclic amino group which is represented by $R_7R_8N$- may together form a bi-cyclic amino group, and (iii) further that said cyclic amino group or said bi-cyclic amino group may contain therein a double bond.

Claims for the following Contracting State: ES

1. A process for preparing a pyridobenzoxazine derivative represented by the following formula (G-H) or a salt thereof,

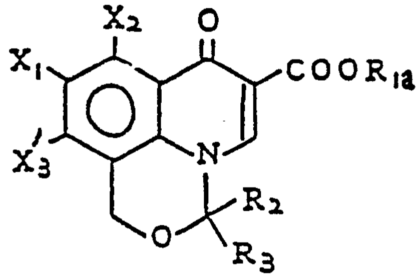

(GH)

wherein $X_1$, $X_2$, and $X'_3$ individually represent a hydrogen atom or a halogen atom; $R_2$ and $R_3$ individually represent a hydrogen atom, a lower alkyl group which may be substituted by halogen atoms, or a lower alkenyl group; or $R_2$ and $R_3$ may, in combination, form a 3-6 membered ring together with the adjacent carbon atom; and $R_{1a}$ represents a hydrogen atom or a lower alkyl group;
which comprises reacting a quinoline derivative having the following formula (F),

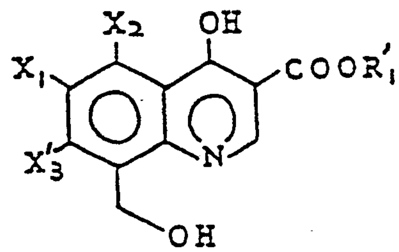

(F)

wherein $X_1$, $X_2$, and $X'_3$ have the same meanings as defined above and $R'_1$ represents a lower alkyl group, and a ketone or aldehyde having the formula, $R_2\underset{\overset{\|}{O}}{C}R_3$,
wherein $R_2$ and $R_3$ have the same meanings as defined above, or a ketal or acetal thereof, and optionally hydrolyzing the compound produced by the reaction.

2. A process for preparing a pyridobenzoxazine derivative represented by the following formula (J) or a salt thereof,

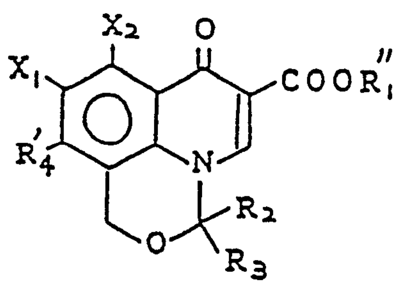

(J)

wherein $X_1$ and $X_2$ individually represent a hydrogen atom or a halogen atom; $R''_1$ represents a hydrogen atom or a lower alkyl group; $R_2$ and $R_3$ individually represent a hydrogen atom, a lower alkyl group which may be substituted by halogen atoms, or a lower alkenyl group; or $R_2$ and $R_3$ may, in combination, form a 3-6 membered ring together with the adjacent carbon atom; $R'_4$ represents a group $R_6S$-, wherein $R_6$ represents a lower alkyl group which may have a substituent, a cyclo-lower alkyl group which may have a substituent, an aryl group which may have a substituent, or a 5 or 6 membered heterocyclic group which may have a substituent; or a group $R_7R_8N$-, wherein $R_7$ and $R_8$ individually represent a hydrogen atom, a lower alkyl group which may have a substituent, a lower alkenyl group which may have a substituent, a cyclo-lower alkyl group which may have a substituent, a lower aralkyl group which may have a substituent, an aryl group which may have a substituent, a 5 or 6 membered heterocyclic group which may have a substituent, or $R_7$ and $R_8$ may, in combination, form a 4-7 membered cyclic amino group together with the adjacent nitrogen atom; provided (i) that the carbon atoms of the cyclic amino group may have one or more

substituents selected from the group consisting of halogen atom, hydroxyl group, alkoxy group, acyloxy group, carboxyl group, alkoxycarbonyl group, aminocarbonyl group which may have a substituent, cyano group, mercapto group, alkylthio group, amino group which may have a substituent, acylamino group which may have a substituent, alkoxycarbonylamino group which may have a substituent, lower alkyl group which may have a substituent, lower alkenyl group which may have a substituent, cyclo-lower alkyl group which may have a substituent, aralkyl group which may have a substituent, and aryl group which may have a substituent, provided that when carbon atoms have two or more substituents which are lower alkyl groups which may have a substituent, the two lower alkyl groups which may have a substituent and the cyclic amino group may together form a bi-cyclic amino group; (ii) that said cyclic amino group or said bi-cyclic amino group may contain in their rings a group $-NR_9$, $-\overset{O}{\overset{\|}{C}}-$, -O-, -S-, -SO-, or $-SO_2-$; wherein $R_9$ is a hydrogen atom, a hydroxyl group, lower alkyl group which may have a substituent, lower alkenyl group which may have a substituent, cyclo-lower alkyl group which may have a substituent, aralkyl group which may have a substituent, aryl group which may have a substituent, acyl group which may have a substituent, a lower alkyloxycarbonyl group which may have a substituent, aralkyloxycarbonyl group which may have a substituent, alkylsulfonyl group which may have a substituent, or arylsulfonyl group which may have a substituent; provided that when $-NR_9-$ is contained in the ring, $R_9$ is a lower alkyl group which may have a substituent, and at least one lower alkyl group which may have a substituent is present on a carbon atom or carbon atoms of the cyclic amino group, one of the lower alkyl groups and said cyclic amino group which is represented by $R_7R_8N-$ may together form a bi-cyclic amino group, and (iii) further that said cyclic amino group or said bi-cyclic amino group may contain therein a double bond; and $R_{1a}$ represents a hydrogen atom or a lower alkyl group;
which comprises reacting a compound of the following formula (I),

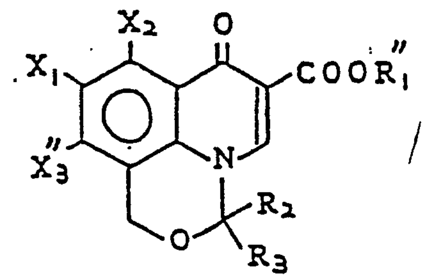

(I)

wherein $X_1$, $X_2$, $R''_1$, $R_2$, and $R_3$ have the same meanings as defined above and $X''_3$ represents a chlorine atom or a fluorine atom,
and a compound represented by the formula $R'_4$-H, wherein $R'_4$ has the same meaning as defined above, or an alkali matal compound thereof.

3. A process for preparing a pyridobenzoxazine derivative represented by the formula (L) or a salt thereof,

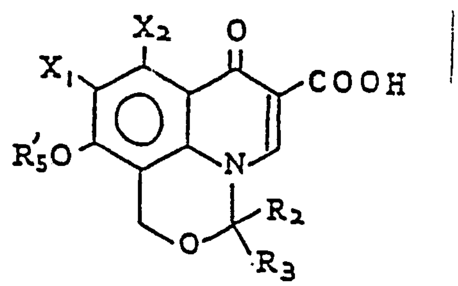

(L)

wherein $X_1$ and $X_2$ individually represent a hydrogen atom or a halogen atom; and $R_2$ and $R_3$ individually represent a hydrogen atom, a lower alkyl group which may be substituted by halogen atoms, or a lower alkenyl group; or $R_2$ and $R_3$ may, in combination, form a 3-6 membered ring together with the adjacent carbon atom; $R'_5$ represents a lower alkyl group which may have a substituent, cyclo-lower alkyl group which may have a substituent, aryl group which may have a substituent, or a 5 or 6 membered heterocyclic

group which may have a substituent;
which comprises reacting a compound having the following formula (K),

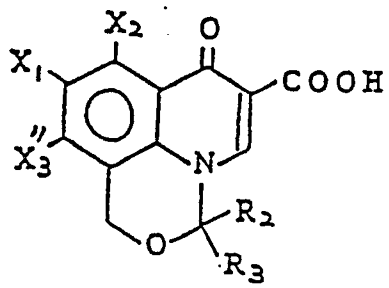

(K)

wherein $X_1$, $X_2$, $R_2$, and $R_3$ have the same meanings as defined above, and $X''_3$ represents a fluorine or chlorine atom;
and a compound represented by the formula $R'_5$-OH, wherein $R'_5$ has the same meaning as defined above, or an alkali matal compound thereof.

4. A process for preparing a pyridobenzoxazine derivative represented by the formula (M) or a salt thereof,

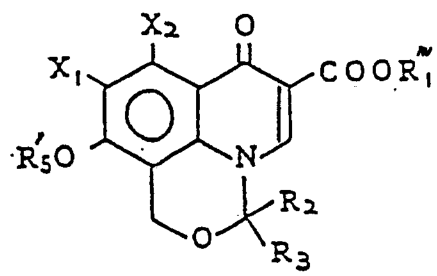

(M)

wherein $X_1$ and $X_2$ individually represent a hydrogen atom or a halogen atom; $R_2$ and $R_3$ individually represent a hydrogen atom, a lower alkyl group which may be substituted by halogen atoms, or a lower alkenyl group; or $R_2$ and $R_3$ may, in combination, form a 3-6 membered ring together with the adjacent carbon atom; $R'_5$ represents a lower alkyl group which may have a substituent, cyclo-lower alkyl group which may have a substituent, aryl group which may have a substituent, or a 5 or 6 membered heterocyclic group which may have a substituent; and $R'''_1$ represents a lower alkyl group;
which comprises reacting a compound having the following formula (L),

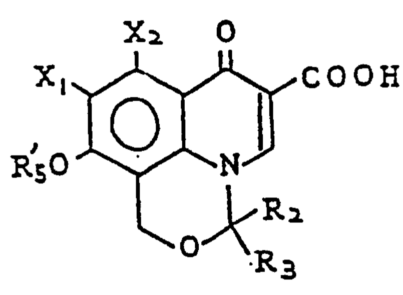

(L)

wherein $X_1$, $X_2$, $R_2$, $R_3$, and $R'_5$ have the same meanings as defined above,
and a compound represented by formula $R'''_1$-X, wherein $R'''_1$ has the same meaning as defined above and X is a halogen atom.

5. A process for preparing a pyridobenzoxazine derivative represented by the formula (N) or a salt thereof,

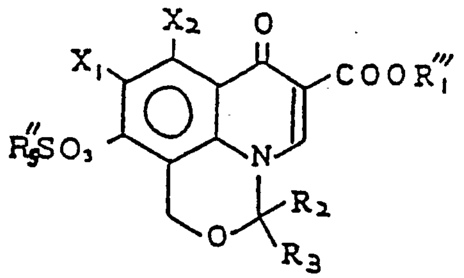

(N)

wherein $X_1$ and $X_2$ individually represent a hydrogen atom or a halogen atom; $R_2$ and $R_3$ individually represent a hydrogen atom, a lower alkyl group which may be substituted by halogen atoms, or a lower alkenyl group; or $R_2$ and $R_3$ may, in combination, form a 3-6 membered ring together with the adjacent carbon atom; $R'''_1$ represents a lower alkyl group; and $R''_5$ represents a lower alkyl group which may have a substituent, cyclo-lower alkyl group which may have a substituent, aryl group which may have a substituent, or a 5 or 6 membered heterocyclic group which may have a substituent;
which comprises reacting a compound having the following formula (M-1),

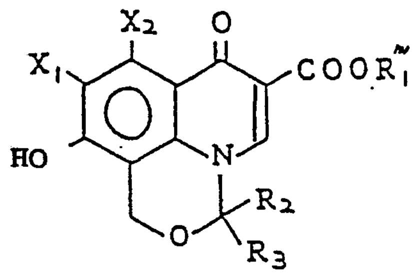

(M-1)

wherein $X_1$, $X_2$, $R_2$, $R_3$, and $R'''_1$ have the same meanings as defined above,
and an alkylsulfonyl halide or an arylsulfonyl halide represented by the formula $R''_5SO_2X$, wherein $R''_5$ has the same meaning as defined above and X is a halogen atom.

6. A process for preparing a pyridobenzoxazine derivative represented by the formula (P) or a salt thereof,

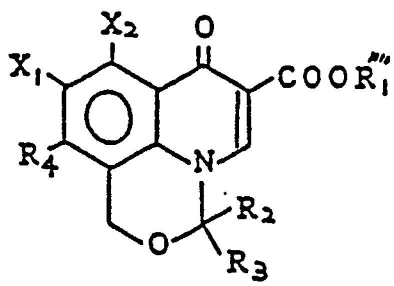

(P)

wherein $X_1$ and $X_2$ individually represent a hydrogen atom or a halogen atom; $R_2$ and $R_3$ individually represent a hydrogen atom, a lower alkyl group which may be substituted by halogen atoms, or a lower alkenyl group; or $R_2$ and $R_3$ may, in combination, form a 3-6 membered ring together with the adjacent carbon atom; $R_4$ represents a hydrogen atom, a hydroxyl group, a group $R_5$-O, wherein $R_5$ represents a lower alkyl group which may have a substituent, a cyclo-lower alkyl group which may have a substituent, an aryl group which may have a substituent, a 5 or 6 membered heterocyclic group which may have a substituent, or an alkyl- or arylsulfonyl group which may have a substituent; a group $R_6S$-, wherein $R_6$ represents a lower alkyl group which may have a substituent, a cyclo-lower alkyl group which may have a substituent, an aryl group which may have a substituent, or a 5 or 6 membered heterocyclic group which may have a substituent; or a group $R_7R_8N$-, wherein $R_7$ and $R_8$ individually represent a hydrogen atom, a lower alkyl group which may have a substituent, a lower alkenyl group which may have a substituent, a cyclo-lower alkyl group which may have a substituent, a lower aralkyl group which may have a substituent,

an aryl group which may have a substituent, a 5 or 6 membered heterocyclic group which may have a substituent, or $R_7$ and $R_8$ may, in combination, form a 4-7 membered cyclic amino group together with the adjacent nitrogen atom; provided (i) that the carbon atoms of the cyclic amino group may have one or more substituents selected from the group consisting of halogen atom, hydroxyl group, alkoxy group, acyloxy group, carboxyl group, alkoxycarbonyl group, aminocarbonyl group which may have a substituent, cyano group, mercapto group, alkylthio group, amino group which may have a substituent, acylamino group which may have a substituent, alkoxycarbonylamino group which may have a substituent, lower alkyl group which may have a substituent, lower alkenyl group which may have a substituent, cyclo-lower alkyl group which may have a substituent, aralkyl group which may have a substituent, and aryl group which may have a substituent, provided that when carbon atoms have two or more substituents which are lower alkyl groups which may have a substituent, the two lower alkyl groups which may have a substituent and the cyclic amino group may together form a bi-cyclic amino group; (ii) that said cyclic amino group or said bi-cyclic amino group may contain in their rings a group $-NR_9$, $-\underset{\underset{O}{\|}}{C}-$, -O-, -S-, -SO-, or $-SO_2-$; wherein $R_9$ is a hydrogen atom, a hydroxyl group, lower alkyl group which may have a substituent, lower alkenyl group which may have a substituent, cyclo-lower alkyl group which may have a substituent, aralkyl group which may have a substituent, aryl group which may have a substituent, acyl group which may have a substituent, a lower alkyloxycarbonyl group which may have a substituent, aralkyloxycarbonyl group which may have a substituent, alkylsulfonyl group which may have a substituent, or arylsulfonyl group which may have a substituent; provided that when $-NR_9-$ is contained in the ring, $R_9$ is a lower alkyl group which may have a substituent, and at least one lower alkyl group which may have a substituent is present on a carbon atom or carbon atoms of the cyclic amino group, one of the lower alkyl groups and said cyclic amino group which is represented by $R_7R_8N-$ may together form a bi-cyclic amino group, and (iii) further that said cyclic amino group or said bi-cyclic amino group may contain therein a double bond; and $R''''_1$ represents a carboxyl protective group;
which comprises reacting a compound of the following formula (O),

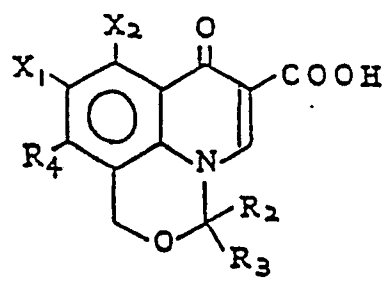

(O)

wherein $X_1$, $X_2$, $R_2$, $R_3$, and $R_4$ have the same meanings as defined above, and a compound represented by the formula $R''''_1X$, wherein $R''''_1X$ has the same meaning as defined above and X is a halogen atom.

European Patent Office

# EUROPEAN SEARCH REPORT

Application Number

EP 89 12 2685

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP-A-0 047 005 (DAIICHI SEIYAKU)<br>* Claim 1 *<br>--- | 1 | C 07 D 498/06<br>A 61 K 31/535//<br>(C 07 D 498/06<br>C 07 D 265:00<br>C 07 D 221:00 ) |
| A | EP-A-0 234 995 (SANOFI)<br>* Claim 1 *<br>--- | 1 | |
| A | CHEMICAL ABSTRACTS, vol. 98, no. 23, 6th June 1983, page 662, abstract no. 198294p, Columbus, Ohio, US; & JP-A-57 203 085 (DAIICHI SEIYAKU CO., LTD) 13-12-1982<br>* Whole document *<br>----- | 1 | |
| | | | **TECHNICAL FIELDS SEARCHED (Int. Cl.5)** |
| | | | C 07 D 498/00<br>A 61 K 31/00 |

The present search report has been drawn up for all claims

| Place of search | Date of completion of the search | Examiner |
|---|---|---|
| THE HAGUE | 07-03-1990 | VOYIAZOGLOU D. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document